(19) 

**Europäisches Patentamt**
**European Patent Office**
**Office européen des brevets**

(11)  **EP 3 797 687 A1**

(12)  **EUROPEAN PATENT APPLICATION**
published in accordance with Art. 153(4) EPC

(43) Date of publication:
**31.03.2021  Bulletin 2021/13**

(21) Application number: **19807340.5**

(22) Date of filing: **21.05.2019**

(51) Int Cl.:
*A61B 5/1455* (2006.01)      *A61B 10/00* (2006.01)
*G01N 21/17* (2006.01)       *G01N 21/27* (2006.01)
*G01N 21/359* (2014.01)      *G01N 21/49* (2006.01)

(86) International application number:
**PCT/JP2019/020142**

(87) International publication number:
**WO 2019/225612 (28.11.2019 Gazette 2019/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.05.2018  JP 2018097589**

(71) Applicants:
• **Medical Photonics Co., Ltd.
  Sapporo-shi, Hokkaido 001-0021 (JP)**
• **Samsung Electronics Co., Ltd.
  Suwon-si 16677 (KR)**

(72) Inventors:
• **TAKAMIZAWA, Atsushi
  Sapporo-shi, Hokkaido 001-0021 (JP)**

• **IINAGA, Kazuya
  Sapporo-shi, Hokkaido 001-0021 (JP)**
• **CHANG, KI YOUNG
  Suwon-si, Gyeonggi-do, 16677 (KR)**
• **LEE, JOON HYUNG
  Suwon-si, Gyeonggi-do, 16677 (KR)**
• **NAMKOONG, KAK
  Suwon-si, Gyeonggi-do, 16677 (KR)**
• **LEE, YEOL HO
  Suwon-si, Gyeonggi-do, 16677 (KR)**
• **JUNG, WON JONG
  Suwon-si, Gyeonggi-do, 16677 (KR)**
• **NAM, JUNG YONG
  Suwon-si, Gyeonggi-do, 16677 (KR)**

(74) Representative: **Vossius & Partner
Patentanwälte Rechtsanwälte mbB
Siebertstrasse 3
81675 München (DE)**

(54)  **BLOOD VESSEL DETECTION DEVICE AND METHOD THEREFOR**

(57)  [Problem] To provide an apparatus and a method that make it possible to identify a blood vessel location. [Solution] The present invention comprises: a radiation unit, which includes a light-blocking plate having a direction adjustment part for enhancing the rectilinearity of radiation light on an irradiated surface, and which irradiates a subject with light of a first wavelength in the absorption band of hemoglobin and with light of a second wavelength that is light of a wavelength the absorption of which by hemoglobin is less than the light of the first wavelength; a light intensity detection unit that detects intensity of light emitted from the subject at one or more locations, the light intensity detection unit being arranged either at a predetermined spacing from a location of light irradiation by the radiation unit or continuously therefrom; and a control unit for calculating scattering information from the light intensity of the light of the second wavelength, calculating absorption information from the light intensity of the light of the first wavelength, calculating blood flow information from the light intensity of the light of the second wavelength, and detecting a blood vessel from the scattering information, the absorption information, and the blood flow information.

FIG. 1

EP 3 797 687 A1

**Description**

Technical Field

The present invention relates to a blood vessel sensing apparatus and a method therefor.

Background Art

Attention has been directed to postprandial hyperlipidemia as a risk factor of the arteriosclerosis. There has been a report stating that an increase in the concentration of neutral lipid in a non-hunger state increases the risk of development of an event of coronary artery diseases.

To diagnose the postprandial hyperlipidemia, it is necessary to observe a change in in-blood lipid concentration for 6 to 8 hours after meals. That is, to measure the state of postprandial hyperlipidemia, it is necessary to place a subject under restraint for 6 to 8 hours and collect blood multiple times. The diagnosis of the postprandial hyperlipidemia is therefore no better than clinical studies, and diagnosing the postprandial hyperlipidemia at a clinical site is not practical.

Patent Literature 1 discloses an approach to a solution of the problem described above. According to the approach disclosed in Patent Literature 1, the noninvasive lipid measurement can eliminate blood collection. The in-blood lipid can therefore be measured not only in a medical institution but at home. Allowing instantaneous data acquisition allows temporally continuous in-blood lipid measurement.

Citation List

Patent Literature

Patent Literature 1: International Publication No. 2014/087825

Summary of Invention

Technical Problem

The approach to measurement of in-blood lipid based on the noninvasive lipid measurement of the related art is directed to a homogeneous system. The noninvasive lipid measurement is, however, measurement of a living body formed of a plurality of tissues, such as skins and muscles. It is therefore conceivable that the homogeneous system theory is not effective in the noninvasive lipid measurement in some cases. In practice, a measured value on a visually recognized vein differs from a measured value in a site where no vein is visually recognized. It is therefore believed that there is an optimum measurement site in the noninvasive lipid measurement.

An object of the present invention, which has been made to solve the problem with the related art, is to provide an apparatus and a method that allow detection of an optimum measurement site for noninvasive measurement of an in-blood component.

Solution to Problem

A blood vessel sensing apparatus according to the present invention includes a radiation unit that includes a light blocking plate including a direction adjuster that is located in a light radiation surface and increases straightness of radiated light, the radiation unit radiating light to a subject, the light formed of light having a first wavelength that falls within a hemoglobin absorption band and light having a second wavelength at which hemoglobin absorbs the light by an amount smaller than an amount by which hemoglobin absorbs the light having the first wavelength, optical intensity detection units that are spaced apart at predetermined gaps or continuously from a position irradiated with the light from the radiation unit and detect optical intensities of light emitted from the subject in one or more positions, and a control unit that calculates scatter information based on an optical intensity of the light having the second wavelength, calculates absorption information based on an optical intensity of the light having the first wavelength, calculates blood flow information based on the optical intensity of the light having the second wavelength, and senses a blood vessel based on the scatter information, the absorption information, and the blood flow information.

A blood vessel sensing method according to the present invention includes radiating light to a subject via a light blocking plate including a direction adjuster for increasing straightness of radiated light, the light formed of light having a first wavelength that falls within a hemoglobin absorption band and light having a second wavelength at which hemoglobin absorbs the light by an amount smaller than an amount by which hemoglobin absorbs the light having the first wavelength, detecting optical intensities of light emitted from the subject in one or more positions spaced apart at predetermined gaps or continuously from a position irradiated with the light, and calculating scatter information based on an optical intensity of the light having the second wavelength, calculating absorption information based on an optical intensity of the light having the first wavelength, calculating blood flow information based on the optical intensity of the light having the second wavelength, and sensing a blood vessel based on the scatter information, the absorption information, and the blood flow information.

Advantageous Effects of Invention

The blood vessel sensing apparatus and method according to the present invention allow improvement in accuracy, preciseness, and other types of precision of a measured value in noninvasive blood measurement.

Brief Description of Drawings

[Figure 1] Figure 1 shows the configuration of a blood vessel sensing apparatus according to an embodiment.
[Figure 2] Figure 2 shows the configuration of a direction adjuster.
[Figure 3] Figure 3 shows the result of examination using a pseudo-living body.
[Figure 4] Figure 4 shows that light is scattered by in-blood lipid.
[Figure 5] Figure 5 shows that light is scattered by in-blood lipid.
[Figure 6] Figure 6 shows the configuration of a control system of a blood vessel sensing apparatus according to the embodiment.
[Figure 7] Figure 7 shows the relationship between the distance from a vein and the intensity.
[Figure 8] Figure 8 is a flowchart of a lipid measurement process in the embodiment.
[Figure 9] Figure 9 shows the relationship between a blood vessel imaged by a vein visualizer and the position marked by using a position determining function.
[Figure 10] Figure 10 shows the relationship between a vein and a position marked by using the position determining function with no direction adjuster used.
[Figure 11] Figure 11 shows examination of the blood vessel position determining function.

Description of Embodiment

A blood vessel sensing apparatus and a method therefor that are an embodiment of the present invention will be described below in detail with reference to the drawings.

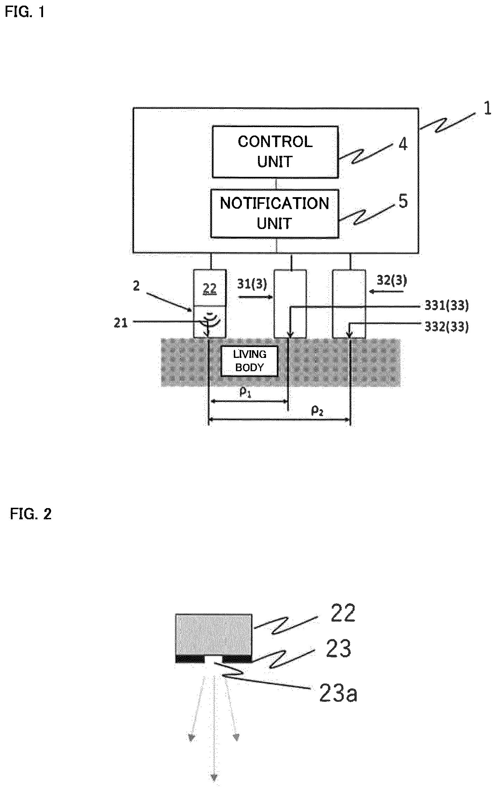

Figure 1 shows the configuration of the blood vessel sensing apparatus according to the embodiment.
A blood vessel sensing apparatus 1 according to the embodiment includes a radiation unit 2, an optical intensity detection unit 3, a control unit 4, and a notification unit 5, as shown in Figure 1.
The irradiation unit 2 includes a light source 22 for radiating light to a predetermined radiation position 21 on a predetermined site of a living body from a point outside the living body toward the interior of the living body. The light source 22 can adjust the wavelength of the radiated light. The light source 22 can adjust the range of the wavelength in such a way that the wavelength range does not fall within the range of the wavelengths at which the light is absorbed by inorganic substances of the blood plasma. The light source 22 can perform the adjustment in such a way that the wavelength range does not fall within the range of the wavelengths at which the light is absorbed by the cell components of the blood. The cell components of the blood used herein are the red blood cells, white blood cells, and platelets in the blood. The inorganic substances of the blood plasma are water and electrolytes in the blood.
The radiation unit 2 in the embodiment can arbitrarily adjust the time length of light radiation, for example, continuous light radiation or pulsed light radiation in accordance with a method for calculating a scatter coefficient $\mu$eff calculated by the control unit 4, which will be described later. The radiation unit 2 can arbitrarily modulate the intensity or phase of the radiated light.
The radiation unit 2 may include a light source 22 that radiates light having a fixed wavelength. The radiation unit 2 may include the combination of a plurality of light sources that radiate light fluxes having different wavelengths or may radiate the combination of light fluxes having a plurality of wavelengths. The radiation unit 2 is, for example, a fluorescent lamp, an LED, a laser, an incandescent lamp, an HID, or a halogen lamp. The illuminance of the light from the radiation unit 2 may be controlled by the control unit 4 or a separately provided control circuit.
In the embodiment, the light source 22 is an LED (light emitting diode). The light source 22 includes a direction adjuster 23 for increasing the straightness of the light radiated from the LED. When an LED is used as it is as the light source 22, diffusion of the radiated light could add an error to a measured value as what is called ambient light could. Further, the radiated light diffuses at the surface of the living body and is therefore affected by substances present between a vein and the light source, such as the skin.

In the embodiment, a method for measuring an optimum depth by controlling the depth to which the radiated light reaches has been examined. The scatter coefficient of a living body tissue, such as the skin of a living body, is assumed to be 1.0/mm, so that it is believed that the light starts scattering when the light reaches the depth of 1 mm. In the embodiment, the direction adjuster 23 including a pinhole 23a having a diameter of 0.8 mm is disposed at the light emitting surface of the LED that forms the light source 22, as shown in Figure 2. The amount of a diffusion component of the light emitted from the LED that forms the light source 22 is thus reduced, whereby the straightness of the light is increased.

The diameter of the pinhole 23a is preferably greater than or equal to 0.4 mm but smaller than or equal to 1.5 mm. When the numerical range described above is employed, incident light with suppressed diffusion is achieved. Causing the light to be radiated via a range smaller than the LED, which is the light source of the incident light, allows reduction in noise due, for example, to the outer frame of the LED device. When the diameter of the pinhole 23a is greater than 1.5 mm, the amount of diffusion component of the light emitted from the LED that forms the light source 22 increases. When the diameter of the pinhole 23a is smaller than 0.4 mm, the incident light intensity necessary for the measurement is not likely to be provided.

The diameter of the pinhole 23a is more preferably greater than or equal to 0.8 mm but smaller than or equal to 1.2 mm. When the numerical range described above is employed, the radiated light becomes pseudo-straight light, which provides an effect approximate to the effect derived from the diffusion theory, which is the measurement principle, and further maintains the optical intensity necessary for the measurement.

The diameter of the pinhole 23a is still more preferably greater than or equal to 0.8 mm but smaller than or equal to 0.9 mm. When the numerical range described above is employed, the radiated light becomes more-pseudo-straight light, which advantageously allows application of a diffusion theory that follows the measurement principle.

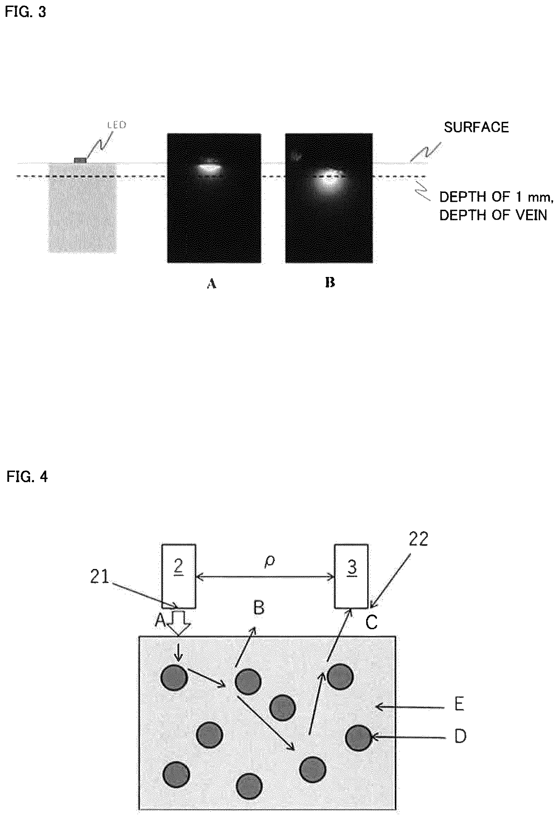

A pseudo-living body having optical characteristics adjusted in the same manner as those of a real living body ($\mu s$=1.0/mm, $\mu a$=0.01/mm) was used to examine the effect of the pinhole 23a. Figure 3 shows the result of the examination. The result shows that the incident light diffuses after penetrating the living body to a depth of about 1 mm (B in Figure 3). On the other hand, when no pinhole is provided, the light diffuses at the surface of the living body (A in Figure 3). The above results show that the light radiated from the LED passes through the pinhole 23a, which increases the straightness of the light, whereby the light can penetrate the living body to a target depth.

The embodiment has been described with reference to the aspect in which the direction adjuster 23 includes the pinhole 23a, but not necessarily. For example, the direction adjuster 23 only needs to have a configuration in which an optical system, such as a lens, can increase the straightness of the light.

The light source 22 in the embodiment radiates light having a first wavelength and light having a second wavelength different from the first wavelength. The light having the first wavelength is light for acquiring absorption information. The light having the second wavelength is light for acquiring scatter information and blood flow information.

The light having the first wavelength for acquiring the "absorption information" may be light having a wavelength that falls within a wavelength band where the light is absorbed by in-blood hemoglobin. For example, the light source 22 preferably radiates visible light as the light having the first wavelength. Using a wavelength that falls within the hemoglobin absorption band allows acquisition of absorption information dependent on hemoglobin. The hemoglobin absorption band ranges from about 400 to 700 nm, which nearly coincides with the wavelength range of visible light. The wavelength range of the light from the light source 22 therefore preferably ranges from 400 to 700 nm.

The light having the second wavelength for acquiring the "scatter information" and "blood flow information" may be light having a wavelength that falls within a wavelength band where the in-blood hemoglobin absorbs the light by an amount smaller than the amount by which the in-blood hemoglobin absorbs the light having the first wavelength. For example, the light source 22 preferably radiates near infrared light as the light having the second wavelength. In addition, near infrared light is absorbed by tissues, such as water, only by a small amount and is therefore readily used to accurately measure information, for example, on blood flow motion.

To acquire "scatter information" and "blood flow information," the range of the wavelength of the light from the light source 22 is preferably formed of the range shorter than or equal to about 1400 nm and the range from about 1500 to 1860 nm in consideration of the range of the wavelengths at which the light is absorbed by the inorganic substances of the blood plasma. Further, the range of the wavelength of the light from the light source 22 is more preferably formed of the range from about 580 to 1400 nm and the range from about 1500 to 1860 nm in consideration of the range of the wavelengths at which the light is absorbed by the cell components of the blood.

The thus set wavelength range employed by the light source 22 suppresses the influence of the light absorption due to the inorganic substances of the blood plasma and the influence of the light absorption due to the cell components of the blood on the light to be detected by the optical intensity detection unit 3, which will be described later. In the thus set wavelength range, no absorption large enough to identify a substance is present, whereby optical energy loss due to the absorption is negligibly small. The light in the blood therefore propagates over a large distance when scattered by lipid in the blood and exits out of the living body.

The light source 22 in the embodiment radiates both visible light and near infrared light. The light source 22 may instead include a plurality of separate light sources formed of a light source that radiates visible light and a light source that radiates near infrared light. When a plurality of light sources are employed, it is unnecessary to distinguish wavelengths from each other when the optical intensity detection unit 3 receives the light. When a plurality of light sources are employed, the plurality of light sources are each preferably provided with a light blocking plate having a pinhole.

The optical intensity detection unit 3 receives light emitted from the living body toward the space outside the living body and detects the optical intensity of the light. In a case where a plurality of optical intensity detection units 3 are used, the optical intensity detection units 3 are disposed at different distances from the radiation position 21 as a rough center. In the embodiment, a first optical intensity detection unit 31 and a second optical intensity detection unit 32 are sequentially arranged from the radiation position 21 along a straight line at a predetermined interval in the same plane, as shown in Figure 1. The optical intensity detection unit 3 may be formed of a photodiode, a CCD, or a CMOS device.

In the embodiment, let $\rho1$ be a first inter-radiation-detection distance from the radiation position 21 to a first detection position 331, where the first optical intensity detection unit 31 detects light, as shown in Figure 1. Similarly, let $\rho2$ be a second inter-radiation-detection distance from the radiation position 21 to a second detection position 332, where the second optical intensity detection unit 32 detects light.

A predetermined distance $\rho$ is set between the radiation position 21, where the living body is irradiated with the light, and a detection position 31, where the intensity of the light emitted from the blood (E in Figure 4) in the living body is detected, as shown in Figure 4. The thus set predetermined distance $\rho$ suppresses the influence of the light directly emitted from the living body (B in Figure 4), which is the radiated light (A in Figure 4) reflected off the surface of the living body and scatterers in the vicinity of the surface. The radiated light reaches the depth where lipid, such as lipoprotein, is present and is then reflected off the lipid (D in Figure 4) in the blood. After the radiated light is reflectively scattered by the lipid, the optical intensity of the resultant backscattered light (C in Figure 4) emitted from the living body is detected. Increasing the distance $\rho$ between the radiation position 21 and the detection position 31 increases the optical path length. The number of times when the light collides with the lipid therefore increases, so that the detected light is greatly affected by the scattering. Increasing the distance $\rho$ as described above allows the influence of the scattering that is small and has therefore been difficult to detect in related art to be readily captured.

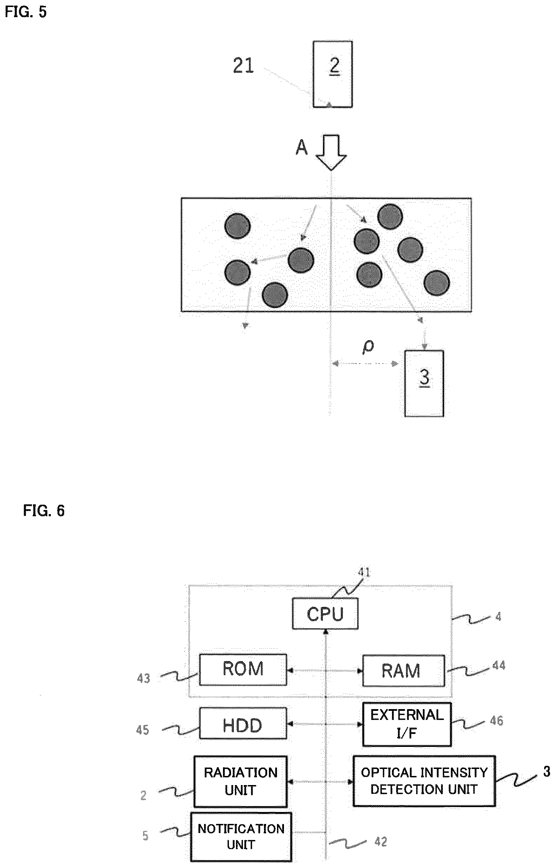

Instead, as shown in the arrangement in Figure 5, the living body (E in Figure 5) may be sandwiched between the radiation unit 2 and the optical intensity detection unit 3, and the optical intensity detection unit 3 may detect the light from the radiation unit 2.

Lipoprotein, which is a measurement target, has a spherical structure covered with apoprotein and other substances. Lipoprotein is present in the form of a solid-like substance in the blood. Lipoprotein is so characterized as to reflect light. In particular, cylomicron (CM), VLDL, and other substances having a large particle diameter and specific gravity contain a large amount of triglyceride (TG) and are so characterized as to be more likely to scatter light. The optical intensity detected by the optical intensity detection unit 3 is therefore affected by the light scattered by lipoprotein.

In the case where a plurality of detection positions 31 are set, the detection positions 31 are not necessarily arranged linearly as long as they are arranged at different distances from the radiation position 21 as a rough center, and a circular arrangement, a wavy arrangement, a zigzag arrangement, or any other arrangement can be selected as appropriate. The first inter-radiation-detection distance $\rho1$ and the second inter-radiation-detection distance $\rho2$, which are each the distance from the radiation position 21 to the detection position 31, and the gap between the detection positions 331 and 332 are not limited to a fixed distance and may instead be continuously changed.

The configuration of a control system of the blood vessel sensing apparatus 1 will next be described. Figure 6 is a block diagram of the blood vessel sensing apparatus 1 according to the embodiment. A CPU (central processing unit) 41, a ROM (read only memory) 43, a RAM (random access memory) 44, a storage unit 45, an external I/F (interface) 46, the radiation unit 2, the optical intensity detection unit 3, and the notification unit 5 are connected to each other via a system bus 42. The CPU 41, the ROM 43, and the RAM 44 form the control unit (controller) 4.

The ROM 43 stores in advance a program executed by the CPU 41 and thresholds used by the CPU 41.

The RAM 44 has an area where the program executed by the CPU 41 is developed, a variety of memory areas, such as a work area where the program processes data, and other areas.

The storage unit 45 stores data, such as sensed and calculated optical intensities and $\mu$eff. The storage unit 45 may be an internal memory that stores information in a nonvolatile manner, such as an HDD (hard disk drive), a flash memory, and an SSD (solid-state drive).

The external I/F 46 is an interface for communication with an external apparatus, for example, a client terminal (PC). The external I/F 46 only needs to be an interface that performs data communication with the external apparatus and may, for example, be an instrument (such as USB memory) locally connected to the external apparatus or a network interface for the communication via a network.

The control unit 4 calculates the scatter information in the living body based on the optical intensity detected by the optical intensity detection unit 3. The scatter information is calculated based on the intensity of the light having a predetermined wavelength radiated from the light source 22 and detected by the optical intensity detection unit 3. In the embodiment, the scatter information is calculated based on the intensity of the light radiated from the light source 22 and detected by the optical intensity detection unit 3 at wavelengths of 810 nm and 970 nm.

The scatter information is information produced when light absorbed by substances in the living body only by a small amount or hardly absorbed at a certain wavelength (second wavelength) is scattered by scatterers. The scatter information depends on the amount of in-blood lipid.

Examples of the scatter information includes the scatter coefficient $\mu$eff. A method for calculating the scatter coefficient $\mu$eff will now be described.

The control unit 4 in the embodiment calculates an optical intensity ratio or an optical intensity difference, as shown in Figure 1.

The control unit 4 calculates the scatter coefficient $\mu$eff from the logarithm of the optical intensities detected in a plurality of positions by the optical intensity detection unit 3. The control unit 4 calculates the scatter coefficient $\mu$eff based on a scattering phenomenon in which the amount of attenuation of the radiated light due to the scattering increases with the distance to a detection position 33.

The radiation unit 2 radiates continuous light having a predetermined optical intensity, and the control unit 4 calculates the scatter coefficient $\mu$eff from the distance $\rho$ between the light radiation unit and the optical intensity detection unit and the product of the square of $\rho$ and the optical intensity R ($\rho$) detected by the first optical intensity detection unit 31 (Numerical Expression 1).

[Numerical Expression 1]

$$\ln\left(\rho^2 R(\rho)\right) = -\mu_{eff}\rho + \ln\frac{S_0}{2\pi}\frac{3\mu_a}{\mu_{eff}}$$

The method for calculating the scatter coefficient $\mu$eff carried out by the control unit 4 is not limited to the calculation method described above.

The control unit 4 calculates the absorption information in the living body based on the optical intensity detected by the optical intensity detection unit 3.

The absorption information depends on hemoglobin and is produced by using a wavelength in the hemoglobin absorption band (first wavelength). The information depending on hemoglobin is used as blood information. The wavelength at which the absorption information used for position determination is acquired is desirably any of the wavelengths of visible light, which falls within the in-blood hemoglobin absorption band. On the other hand, near infrared light is desirable to check the blood flow information. Near infrared light is absorbed by tissues, such as water, only by a small amount and is therefore readily used to accurately measure information, for example, on blood flow motion.

The absorption information is the intensity of the light having a predetermined wavelength radiated from the light source 22 and detected by the optical intensity detection unit 3. In the embodiment, the absorption information is the intensity of the light radiated from the light source 22 and detected by the optical intensity detection unit 3 at a wavelength of 660 nm.

The blood that is the measurement target flows through a blood vessel, unlike, for example, the skin tissue. A dynamic parameter produced by the blood flow is defined as the blood flow information. In the embodiment, the blood flow information is calculated by measuring the dynamic parameter for a fixed length of time in preparation for analysis to determine the position of the blood vessel.

The blood flow information is information produced when light absorbed by substances in the living body only by a small amount or hardly absorbed at a certain wavelength (second wavelength) is scattered by scatterers.

The control unit 4 calculates the blood flow information, which is an index for the measurement of the motion of the blood by analysis using a standard deviation, Brownian motion, an autocorrelation function, frequency analysis, speckles, Doppler shift, Reynold's number, the amount of blood flow, the amount of blood, pulsation width, and other factors. The control unit 4 may calculate the blood flow information by using the amount of change in the optical intensity over an optical intensity measurement length of time shorter than or equal to 20 sec.

To measure a measurement target site in related art, attention is not directed to the amount of change with time in a measured value, but the average of the changes is employed. In the blood measurement, however, measurement of a site where the blood is abundant or dense, such a vein, allows a large number of pieces of information on the blood to be provided, whereby the number of noise factors decreases. In the noninvasive measurement, to evaluate whether incident light has passed through a vein, it is desirable to acquire information provided from the blood flow.

To measure heart beat periodicity, such as the pulse, however, it is believed that an artery is desirably measured. Therefore, to determine the position of a vein that is the measurement target, it is desirable to measure variation in temporal change due to the blood flow in the received optical intensity for a fixed length of time.

That is, to observe a pulsation period (from about 0.5 to 2.0 Hz), it can be said that the skin layer is a living body site appropriate for the lipid measurement. On the other hand, a non-periodic blood flow information showing no pulsation period is information representing the position of a vein (at least depending on vein information), and it can therefore be said that a vein is a living body site appropriate for the lipid measurement.

To distinguish the two types of information described above from each other, the sampling rate employed by the optical intensity detection unit is desirably 10 msec or shorter, and the resolution of the optical intensity detection unit is desirably at least 16 bits.

Examples of the blood flow information include a variation coefficient CV of the scatter coefficient $\mu$eff.

The control unit 4 calculates the variation coefficient CV of the scatter coefficient $\mu$eff based on a temporal change in the calculated scatter coefficient $\mu$eff. The variation coefficient CV can be calculated, for example, by using Expression 2 below.

[Numerical Expression 2]

$$X = \frac{\sigma}{\bar{x}}$$

x: Measured value of optical intensity
$\bar{x}$: Average of measured values of optical intensity
$\sigma$: Standard deviation of optical intensity
X: Turbulence for fixed length of time = variation coefficient CV of the scatter coefficient $\mu$eff

The standard deviation of the optical intensity is determined by Expression 3 below.

[Numerical Expression 3]

$$\sigma = \sqrt{\langle x^2 \rangle - \bar{x}^2}$$

In Expression 3, <> represents the average.

To calculate the variation coefficient CV, the length of time for which the scatter coefficient $\mu$eff is measured may be longer than or equal to 1 msec but shorter than or equal to 30 sec, preferably, longer than or equal to 5 msec but shorter than or equal to 25 sec, more preferably, longer than or equal to 10 msec but shorter than or equal to 20 sec ("sec" is abbreviation for "second").

The control unit 4 calculates the scatter information, the absorption information, and blood flow information to determine the position of a blood vessel based on the calculated scatter information, absorption information, and blood flow information.

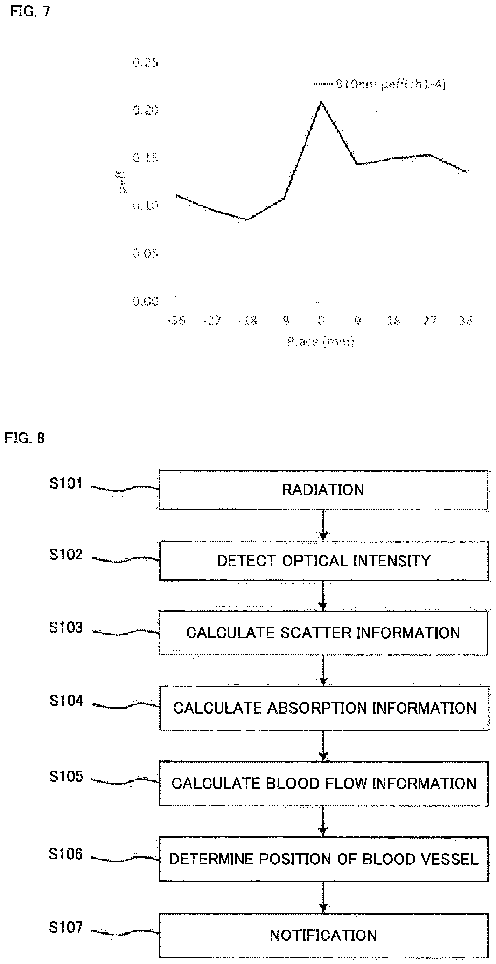

The embodiment provides an apparatus and a method for searching for the position of a blood vessel in a two-dimensional (planar) manner. The degree of scattering at a vein is greater than that at the other sites, as shown in Figure 7, whereby it is expected that the position where the scattering occurs is detected as the position of the blood vessel. However, the strength of scattering, which is also related to the thickness and depth of the blood vessel, cannot be unconditionally measured based only on absorption. Actual comparison of the position determining function between a case where the lipid measurement provides a satisfactory result and a case where the lipid measurement provides an unsatisfactory result shows that the variation in the received optical intensity differs between the two cases. It is speculated that the difference reflects the blood flow in the vein. The blood flow information is degraded, for example, by the skin layer when the location of the measurement is too deep with respect to optimum measurement conditions.

In the embodiment, an LED is used, parallelized light having straightness increased by using a pinhole or a lens is incident on a living body, and the absorption information and the blood flow information on the blood are combined with each other, whereby the position of a blood vessel can be sensed in advance.

The blood that is the measurement target flows through a blood vessel, unlike, for example, the skin tissue. In the embodiment, the blood flow information is calculated by measuring the blood for a fixed length of time in preparation for the analysis. Further, the absorption information representing the entire scattering present in the optical path is calculated. The position of the blood vessel on an individual basis is then determined from the two parameters.

The absorption information and the blood flow information are not necessarily acquired via a communication line and may be manually input.

The control unit 4 senses as the scatter information that $\mu$eff ($\mu$eff stands for effective scatter coefficient, the same holds true in the following description) at the wavelength of 810 nm is maximized.

The wavelength of 810 nm is the wavelength of the light radiated from the light source 22 for blood flow information acquisition. The information on $\mu$eff at the wavelength is information dependent on the in-blood turbidity.

The scatter coefficient $\mu$eff can be determined by using Numerical Expression 1 described above based on the optical intensities detected by the first optical intensity detection unit 31 and the second optical intensity detection unit 32.

In Numerical Expression 1, $\rho$ is the distance from the radiation position, and R($\rho$) is the optical scattering intensity at the distance (value measured with detection photodiode in the present apparatus). When these parameters described above are applied to Numerical Expression 1, the gradient in the expression described above is $\mu$eff.

In the measurement over an arbitrary range of a subject, when the optical intensity is detected multiple times, the value of $\mu$eff changes in accordance with the measurement position. The control unit 4 compares the measured $\mu$eff with the preceding value of $\mu$eff and replaces the saved value of $\mu$eff when the measured $\mu$eff is greater than the preceding value of $\mu$eff. As a result, the control unit 4 automatically stores the maximum value of $\mu$eff at the wavelength of 810 nm within the position search range.

The control unit 4 senses that $\mu$eff as the scatter information is maximized at 970 nm.

The wavelength of 970 nm is the wavelength of the light radiated from the light source 22 for blood flow information acquisition. The information on $\mu$eff at the wavelength is information dependent on the in-blood turbidity.

The scatter coefficient $\mu$eff can be determined by using Numerical Expression 2 described above based on the optical intensities detected by the first optical intensity detection unit 31 and the second optical intensity detection unit 32. How to determine $\mu$eff has been described above.

In the measurement over an arbitrary range of the subject, when the optical intensity is detected multiple times, the value of $\mu$eff changes in accordance with the measurement position. The control unit 4 compares the measured $\mu$eff with the preceding value of $\mu$eff and replaces the saved value of $\mu$eff when the measured $\mu$eff is greater than the preceding value of $\mu$eff. As a result, the control unit 4 automatically stores the maximum value of $\mu$eff at the wavelength of 970 nm within the position search range.

The control unit 4 senses that 660nmAD/810nmCV is minimized.

The value 660nmAD is the absorption information. Specifically, 660nmAD is the value measured by the first optical intensity detection unit 31 or the second optical intensity detection unit 32, a closer one in terms of distance, when the light having the wavelength of 660 nm is radiated.

The value 810nmCV is the blood flow information. The value 810nmCV is the variation coefficient CV of the scatter coefficient $\mu$eff in the case where the wavelength of the radiated light is 810 nm.

The wavelengths of 810 nm and 970 nm are selected as an optimum wavelengths based on the particle size of the scatterers in the light scattering measurement. The wavelength of 660 nm is the wavelength for hemoglobin absorption detection.

In the measurement over an arbitrary range of the subject, when the optical intensity is detected multiple times, the value of 660nmAD/810nmCV changes in accordance with the measurement position. The control unit 4 compares the measured 660nmAD/810nmCV with the preceding value of 660nmAD/810nmCV and replaces the saved value of 660nmAD/810nmCV when the measured 660nmAD/810nmCV is greater than the preceding value of 660nmAD/810nmCV. As a result, the control unit 4 automatically stores the minimum value of 660nmAD/810nmCV within the position search range.

When $\mu$eff as the scatter information is maximized at 810 nm, $\mu$eff as the scatter information is maximized at 970 nm, and the ratio between the absorption information and the blood flow information 660nmAD/810nmCV is minimized, the control unit 4 determines the location under measurement is the blood vessel position where satisfactory data can be acquired in the noninvasive lipid measurement.

The notification unit 5 in the embodiment is, for example, a buzzer, a vibrator, or a lamp. When the control unit 4 determines that blood vessel under measurement is a site appropriate for sensing, the control unit 4 causes the notification unit 5 to cause a buzzer to issue sound, a vibrator to vibrate, or a lamp to emit light. The notification unit 5 notifies a user that the position of the blood vessel is located.

The blood vessel sensing apparatus 1 having the configuration described above carries out a blood vessel sensing process based on a program set in advance. Figure 8 is a flowchart of the blood vessel sensing process in the embodiment.

The radiation unit 2 radiates continuous light to the radiation position 21 via the light blocking plate including the direction adjuster for increasing the straightness of the radiated light (step 101).

The first optical intensity detection unit 31 detects the optical intensity in the first detection position 331, and the second optical intensity detection unit 32 detects the optical intensity in the second detection position 332 (step 102).

The control unit 4 calculates the scatter information in the living body based on the optical intensities detected by the optical intensity detection unit 3 (step 103).

For example, the control unit 4 calculates the scatter coefficient $\mu$eff from the logarithm of the optical intensities detected in the plurality of positions by the optical intensity detection unit 3. The control unit 4 calculates the scatter coefficient μeff based on the scattering phenomenon in which the amount of attenuation of the radiated light due to the scattering increases with the distance to the detection position 33.

The control unit 4 calculates the optical intensity difference or the optical intensity ratio between a first optical intensity in the first detection position 331 and a second optical intensity in the second detection position 332 and calculates the absorption information based on the optical intensity difference or the optical intensity ratio. Instead, the control unit 4 calculates the absorption information from the optical intensity in the first detection position 331 or the optical intensity in the second detection position 332 (step 104).

The control unit 4 calculates the blood flow information, which is an index of the blood flow, from a temporal change in the absorption information (step 105). The control unit 4 may set the length of time of the optical intensity measurement at 20 sec or shorter and calculate the blood flow information from the amount of change in the optical intensity within the length of time of the measurement.

The control unit 4 determines that the predetermined site of the living body irradiated with the light is the position of a blood vessel based on the scatter information, the absorption information, and the blood flow information (step 106).

For example, when μeff is maximized at 810 nm, μeff is maximized at 970 nm, and 660nmAD/810nmCV (with respect to μeff) is minimized, the control unit 4 determines the location under measurement is the blood vessel position. The methods for calculating the maximum of μeff at 810 nm, the maximum of μeff at 970 nm, and the minimum of 660nmAD/810nmCV have been described above.

When the control unit 4 determines that the predetermined site is the position of the blood vessel, the control unit 4 causes the notification unit 5 to cause a buzzer to issue sound, a vibrator to vibrate, or a lamp to emit light (step 107).

As described above, the blood vessel sensing apparatus and method according to the present embodiment allow evaluation of whether or not a position under measurement is the position of a blood vessel based on the absorption information and the blood flow information.

Example

An example of the present invention will be described below, but the present invention is not limited to the example described below.

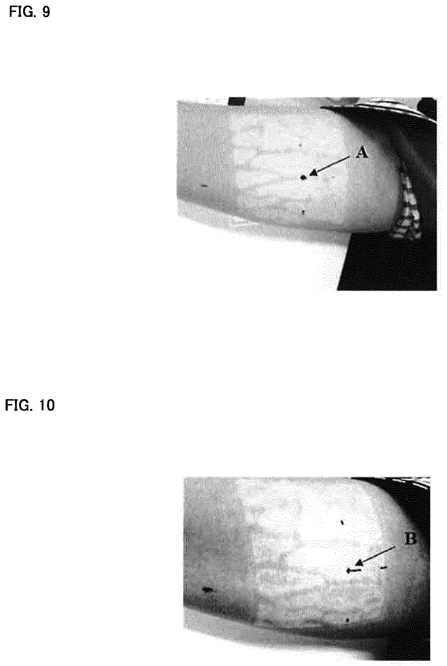

A commercially available vein visualizer was used to examine the blood vessel position determining function in the present example. First, the blood vessel sensing apparatus was used to mark a location that falls within a reference range in the present example, and the location was examined by using the vein visualizer. Figure 9 shows the result of the examination. In Figure 9, the black point (A in Figure 9) at the center of the area defined by four points represents the marked position. Figure 9 shows that the position of a vein was correctly detected.

Figure 10 shows a case where the position determining function was applied with no pinhole used. The position of the blood vessel was not accurately detected (B in Figure 10), as compared with Figure 9.

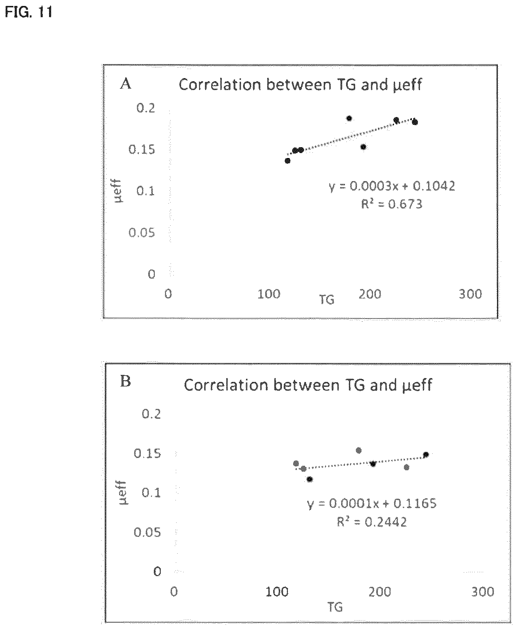

The portion A in Figure 11 shows the result of lipid measurement in the position determined by using the position determining function, and the portion B in Figure 11 shows the result of the lipid measurement in another position. The portion A shows that the correlation coefficient is 0.82, which is satisfactory as compared with the correlation coefficient of 0.49 in the case of B.

The technology described above can also be used to search for the position of a vein or an artery.

The light receiver can be formed, for example, of an array of light receiving elements, a CCD camera, or a CMOS camera to allow two-dimensional information acquisition, and a program can be used to automatically detect an optimum position where the conditions 1 and 2 described above are satisfied.

An embodiment has been described above, and the embodiment is presented by way of example and is not intended to limit the scope of the present invention. The novel embodiment can be implemented in a variety of other forms, and a variety of omissions, replacements, and changes can be made to the embodiment to the extent that they do not depart from the substance of the present invention. The embodiment and variations thereof are encompassed in not only the scope and substance of the present invention but the invention set forth in the claims and the scope equivalent thereto.

Reference Sings list

1    Blood vessel sensing apparatus
2    Radiation unit
3    Optical intensity detection unit
4    Control unit

5    Notification unit

**Claims**

1.  A blood vessel sensing apparatus comprising:

    a radiation unit that includes a light blocking plate including a direction adjuster that is located in a light radiation surface of the radiation unit and increases straightness of radiated light from the radiation unit, the radiation unit radiating light to a subject, the light formed of light having a first wavelength that falls within a hemoglobin absorption band and light having a second wavelength at which hemoglobin absorbs the light by an amount smaller than an amount by which hemoglobin absorbs the light having the first wavelength;
    an optical intensity detection unit that is spaced apart at predetermined gaps or continuously from a position irradiated with the light from the radiation unit and detects optical intensities of light emitted from the subject in one or more positions; and
    a control unit that calculates scatter information based on an optical intensity of the light having the second wavelength, calculates absorption information based on an optical intensity of the light having the first wavelength, calculates blood flow information based on the optical intensity of the light having the second wavelength, and senses a blood vessel based on the scatter information, the absorption information, and the blood flow information.

2.  The blood vessel sensing apparatus according to claim 1, wherein the light having the first wavelength is visible light, and the light having the second wavelength is near infrared light.

3.  The blood vessel sensing apparatus according to claim 1 or 2, wherein the control unit determines that the blood vessel is sensed when the scatter information has a maximum value and a quotient of operation of dividing the absorption information by the blood flow information is a minimum value.

4.  The blood vessel sensing apparatus according to any of claims 1 to 3,
    wherein the scatter information is a scatter coefficient, and
    the control unit calculates the optical scatter coefficient in the subject based on a ratio or a difference between the optical intensities detected in the plurality of positions by the optical intensity detection unit.

5.  The blood vessel sensing apparatus according to any of claims 1 to 4, wherein the control unit calculates the absorption information based on the intensities of the radiated light having the first wavelength and detected by the optical intensity detection unit.

6.  The blood vessel sensing apparatus according to claim 4,
    wherein the blood flow information is a variation coefficient, and
    the control unit calculates the variation coefficient based on a change in the scatter coefficient over a predetermined length of time.

7.  The blood vessel sensing apparatus according to claim 6, wherein the predetermined length of time is longer than or equal to 10 msec but shorter than or equal to 20 sec.

8.  The blood vessel sensing apparatus according to any of claims 1 to 7, wherein the direction adjuster includes a pinhole or a lens.

9.  The blood vessel sensing apparatus according to claim 8, wherein a diameter of the pinhole is greater than or equal to 0.4 mm but smaller than or equal to 1.5 mm.

10. A blood vessel sensing method comprising:

    radiating light to a subject via a light blocking plate including a direction adjuster for increasing straightness of radiated light, the light formed of light having a first wavelength that falls within a hemoglobin absorption band and light having a second wavelength at which hemoglobin absorbs the light by an amount smaller than an amount by which hemoglobin absorbs the light having the first wavelength;
    detecting optical intensities of light emitted from the subject in one or more positions spaced apart at predetermined gaps or continuously from a position irradiated with the light; and

calculating scatter information based on an optical intensity of the light having the second wavelength, calculating absorption information based on an optical intensity of the light having the first wavelength, calculating blood flow information based on the optical intensity of the light having the second wavelength, and sensing a blood vessel based on the scatter information, the absorption information, and the blood flow information.

FIG. 1

FIG. 2

FIG. 3

LED

SURFACE

DEPTH OF 1 mm,
DEPTH OF VEIN

A          B

FIG. 4

21

22

2          ρ          3

A          B          C

E

D

FIG. 5

FIG. 6

FIG. 7

FIG. 8

| | |
|---|---|
| S101 | RADIATION |
| S102 | DETECT OPTICAL INTENSITY |
| S103 | CALCULATE SCATTER INFORMATION |
| S104 | CALCULATE ABSORPTION INFORMATION |
| S105 | CALCULATE BLOOD FLOW INFORMATION |
| S106 | DETERMINE POSITION OF BLOOD VESSEL |
| S107 | NOTIFICATION |

FIG. 9

FIG. 10

FIG. 11

A    Correlation between TG and μeff

$y = 0.0003x + 0.1042$
$R^2 = 0.673$

B    Correlation between TG and μeff

$y = 0.0001x + 0.1165$
$R^2 = 0.2442$

## INTERNATIONAL SEARCH REPORT

International application No.

PCT/JP2019/020142

**A. CLASSIFICATION OF SUBJECT MATTER**
Int.Cl.   A61B5/1455(2006.01)i, A61B10/00(2006.01)i, G01N21/17(2006.01)i,
G01N21/27(2006.01)i, G01N21/359(2014.01)i, G01N21/49(2006.01)i

According to International Patent Classification (IPC) or to both national classification and IPC

**B. FIELDS SEARCHED**

Minimum documentation searched (classification system followed by classification symbols)
Int.Cl. A61B5/06-5/22, A61B9/00-10/06, G01N21/00-21/01, G01N21/17-21/61

Documentation searched other than minimum documentation to the extent that such documents are included in the fields searched
Published examined utility model applications of Japan     1922–1996
Published unexamined utility model applications of Japan   1971–2019
Registered utility model specifications of Japan       1996–2019
Published registered utility model applications of Japan   1994–2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2017-113461 A (TOSHIBA CORP.) 29 June 2017, paragraphs [0001]-[0304], fig. 1-23 (Family: none) | 1-10 |
| A | WO 2016/208010 A1 (HAMAMATSU PHOTONICS K.K.) 29 December 2016, paragraphs [0001]-[0058], fig. 1-12 & US 2018/0180539 A1, paragraphs [0001]-[0088], fig. 1-12 & EP 3315943 A1 | 1-10 |
| A | JP 2013-544151 A (GIVEN IMAGING LTD.) 12 December 2013, paragraphs [0001]-[0111], fig. 1-9 & US 2013/0231536 A1, paragraphs [0001]-[0118], fig. 1-9 & WO 2012/066553 A1 & CN 103209632 A | 1-10 |

☒   Further documents are listed in the continuation of Box C.     ☐   See patent family annex.

| | |
|---|---|
| \*   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 06 August 2019 (06.08.2019) | 13 August 2019 (13.08.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2019/020142

C (Continuation). DOCUMENTS CONSIDERED TO BE RELEVANT

| Category* | Citation of document, with indication, where appropriate, of the relevant passages | Relevant to claim No. |
|---|---|---|
| A | WO 2017/199492 A1 (MEDICAL PHOTONICS CO., LTD.) 23 November 2017, paragraphs [0001]-[0179], fig. 1-14 & EP 3459457 A1, paragraphs [0001]-[0178], fig. 1-13 & CN 109152558 A & KR 10-2019-0008357 A | 1-10 |
| A | WO 2017/141895 A1 (MEDICAL PHOTONICS CO., LTD.) 24 August 2017, paragraphs [0001]-[0133], fig. 1-19 & US 2019/0046091 A1, paragraphs [0001]-[0170], fig. 1-19 & EP 3417779 A1 & CN 108697388 A & KR 10-2018-0111956 A | 1-10 |
| A | WO 2017/119130 A1 (MITSUBISHI CHEMICAL HOLDINGS CORP.) 13 July 2017, paragraphs [0001]-[0044], fig. 1-8 (Family: none) | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014087825 A